Europäisches Patentamt

European Patent Office

Office européen des brevets

(11) Publication number: 0 281 356 B1

# EUROPEAN PATENT SPECIFICATION

(45) Date of publication of patent specification: 15.12.93   (51) Int. Cl.5: **C12N 15/76**

(21) Application number: 88301763.4

(22) Date of filing: 01.03.88

(54) DNA segment conferring high frequency transduction of recombinant DNA vectors.

(30) Priority: 02.03.87 US 20807

(43) Date of publication of application:
07.09.88 Bulletin 88/36

(45) Publication of the grant of the patent:
15.12.93 Bulletin 93/50

(84) Designated Contracting States:
BE CH DE ES FR GB GR IT LI NL SE

(56) References cited:

JOURNAL OF BACHTERIOLOGY, vol. 159, no. 2, August 1984 (Baltimore, USA) K. L. COX et al. "Restriction of Bacteriophag Plaque Formation in Streptomyces spp."

(73) Proprietor: ELI LILLY AND COMPANY
Lilly Corporate Center
Indianapolis Indiana 46285(US)

(72) Inventor: Baltz, Richard Henry
4541 Washington Blvd.
Indianapolis Indiana 46205(US)
Inventor: McHenney, Margaret Ann
5716A Brendon Way West Drive
Indianapolis Indiana 46226(US)

(74) Representative: Hudson, Christopher Mark et al
Erl Wood Manor
Windlesham Surrey GU20 6PH (GB)

**Description**

The present invention relates to transducible vectors that utilize a segment of phage FP43 DNA that enables transduction of the vectors by phage FP43. Phage FP43 does not have cohesive ends, so the transduction system of the present invention differs from the λ system for E. coli, the SP02 system for Bacillus subtilis, and the R4 system for S. lividans. The present invention also relates to a method of transferring DNA into a host cell that involves the use of transducing vectors. The method allows efficient transfer of DNA between many species of Streptomyces and transduction of several other general of actinomycetes.

The figures described below and accompanying the specification are drawn to scale; however, observed restriction fragment size may vary somewhat from calculated size based on map distances. For some restriction enzymes, such as MboI, only certain cut sites are shown for convenience.

Figure 1 is a restriction site and function map of plasmid pIJ702.

Figure 2 is a restriction site and function map of plasmid pRHB101.

Figure 3 is a restriction site and function map of plasmid pRHB106.

Figure 4 is a graph showing the effects of phage FP43 concentration on transduction.

The present invention relates to vectors and methods for a phage-mediated transduction system not only for Streptomyces but also for other organisms throughout the Actinomycetales family, such as Chainia, Saccharopolyspora, and Streptoverticillium. The transduction system utilizes a segment of DNA from bacteriophage FP43 cloned into illustrate plasmids pIJ702 (see Katz et al., 1982, J. Gen. Microbiol. 192: 2703-2714) and pMT660 (see Birch and Cullum, 1985, J. Gen. Microbiol. 131: 1299-1303). As disclosed by Cox and Baltz, 1984, J. Bacteriol. 159: 499-504, FP43 is a temperate bacteriophage with broad host specificity for Streptomyces spp.

The present invention provides transducible cloning vectors for use in Streptomyces and other host cells. The development and exploitation of recombinant DNA technology in Streptomyces depends upon the availability of suitable cloning vectors. This development has been somewhat retarded by the low number of suitable vectors presently available for use in Streptomyces and other antibiotic-producing organisms and the difficulties encountered in developing protoplast transformation procedures. The present invention is useful and especially important in that it expands the number of vectors and hosts suitable for such use.

The vectors of the present invention are particularly useful, because the vectors are small, versatile, and can be transformed and selected in all Streptomyces species and many other heretofore untransformable organisms. Streptomyces provides over half of the clinically important antibiotics and thus is a commercially significant group. The present invention provides new and useful cloning systems and vectors for this industrially important group and allows for the cloning of genes both for increasing the yields of known antibiotics and also for producing new antibiotics and antibiotic derivatives.

For purposes of the present invention, as disclosed and claimed herein, the following terms are defined below.

Antibiotic - a substance produced by a micro-organism which, either naturally or with limited modification, will inhibit the growth of or kill another microorganism or eukaryotic cell.

Antibiotic Biosynthetic Gene - a DNA segment that encodes an enzymatic or other activity that is necessary in the process of converting primary metabolites into antibiotics.

Antibiotic Biosynthetic Pathway - the entire set of antibiotic biosynthetic genes necessary for the process of converting primary metabolites into antibiotics.

Antibiotic Resistance-Conferring Gene - a DNA segment that encodes an enzymatic or other activity that confers resistance to an antibiotic.

ApR - the ampicillin-resistant phenotype or gene conferring same.

cos - a specific phage cohesive and sequence that is cut during packaging of the phage DNA.

Genetic Library or Genomic Library - a set of recombinant DNA cloning vectors into which segments of DNA, comprising substantially all of the DNA of a particular organism or phage, have been cloned.

hft - high frequency transduction, also used to denote a segment of phage DNA that confers high frequency transducibility to a vector.

Infection - the process of phage replication, wherein the phage attaches to and injects its DNA into the host cell, which then supports phage replication and maturation and ultimately release of phage particles through lysis.

mel - the tyrosinase gene.

Recombinant DNA Cloning Vector--any autonomously replicating or integrating agent, including, but not limited to, plasmids, comprising a DNA molecule to which one or more additional DNA segments can be or have been added.

Restriction Fragment--any linear DNA molecule generated by the action of one or more restriction enzymes.

Selectable Marker - a segment of DNA incorporated into a recombinant DNA vector that allows for the identification of cells containing the vector, whether freely replicating or integrated. Selectable markers include antibiotic resistance-conferring genes and other genes such as the tyrosinase or $\beta$-galactosidase genes.

Sensitive Host Cell--a host cell that cannot grow in the presence of a given antibiotic without a DNA segment that provides resistance thereto.

TcR - the tetracycline-resistant phenotype or gene conferring same.

Transduction - the phage-mediated introduction of DNA into a recipient host cell that changes the genotype and results in a change in the recipient cell.

Transductant - a recipient host cell that has undergone transduction.

Transformant - a recipient host cell that has undergone transformation.

Transformation--the introduction of DNA into a recipient host cell that changes the genotype and results in a change in the recipient cell.

tsrR - the thiostrepton-resistant phenotype or gene conferring same.

The present invention provides a method for isolating sequences of actinophage (a phage that infects organisms in the family Actinomycetales) DNA that confer high frequency transduction to recombinant vectors. These phage sequences, denoted as hft sequences, can be isolated by: (1) making a genomic library of the actinophage DNA; (2) transforming the genomic library into a host that supports actinophage infection; (3) infecting the cell transformed in step (2) with the actinophage and collecting the resulting lysate; (4) using the lysate to transduce an organsim of the family Actinomycetales; and (5) isolating the vectors that transduce at high frequency in step (4). This method is especially preferred for isolating hft sequences from Streptomyces phages. The method is illustrated by demonstrating the isolation of the hft sequence of the Streptomyces phage FP43.

Vectors constructed with an hft sequence isolated by the method of the present invention are especially useful in a method of transferring DNA encoding a useful substance to an actinomycetes. This method comprises transducing an actinomycetes with a recombinant DNA vector that comprises: (1) an hft sequence; (2) DNA encoding a useful substance; (3) a plasmid origin of replication; and (4) a selectable marker. Using this method, one can introduce a variety of DNA molecules into an actinomycetes host cell. The DNA molecules can encode such useful substances as antibiotic biosynthetic enzymes, intact antibiotic biosynthetic genes or pathways, and pharmacologically active substances, such as growth hormone, insulin, interferon, and the like.

The present invention also comprises a DNA segment of bacteriophage FP43 that can be used to construct recombinant DNA expression vectors that are transducible at high frequency in a wide variety of organisms. Bacteriophage FP43 can be isolated from Streptomyces griseofuscus(FP43) by the procedure described in Example 1. S. griseofuscus(FP43) contains lysogenized FP43 phage and is available from the Agricultural Research Service, Northern Regional Research Center (NRRL), Peoria, IL 61604, under the accession number NRRL 18184.

The segment of bacteriophage FP43 DNA that confers high frequency transducibility (hft) can be isolated readily and used to construct vectors of the present invention. Illustrative vector pRHB101 was constructed by inserting the ~7.8 kb SphI restriction fragment of phage FP43 into SphI-digested plasmid pIJ702. The ~7.8 kb SphI restriction fragment of phage FP43 comprises the hft sequence.

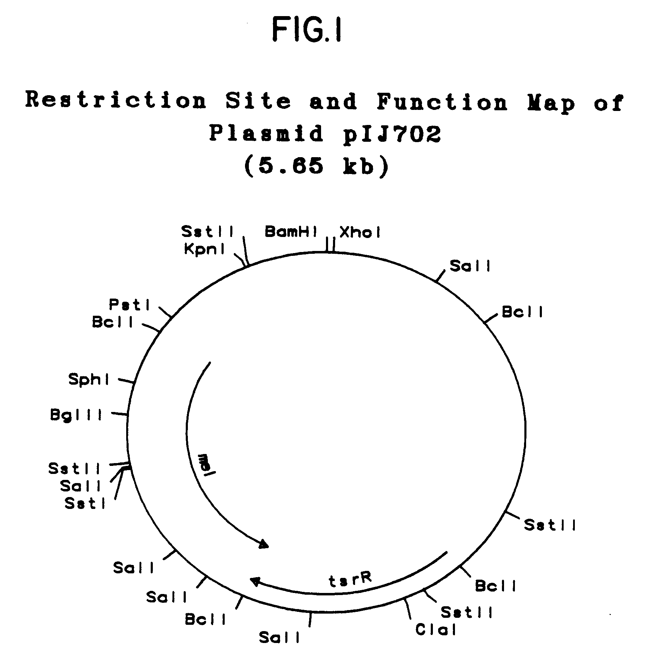

Plasmid pIJ702 is a multicopy plasmid about 5.8 kb in size that has broad host specificity for Streptomyces (see Acebal et al., 1986, FEMS Microbiol. Lett. 35: 79-82; Katz et al., 1982, J. Gen. Microbiol. 192: 2703-2714; Lampel and Strohl, 1986, Appl. Environ. Microbiol. 51: 126-131; and Matsushima and Baltz, 1985, J. Bacteriol. 163: 180-185) and also replicates in Amycolatopsis orientalis, Saccharopolyspora erythraea (see Yamomoto et al., 1986, J. Antibiol. 39: 1304-1313), and Thermomonospora fusca (see Pidcock et al., 1985, Appl. Environ. Microbiol. 50: 693-695). Plasmid pIJ702 was derived from the multicopy, broad host range plasmid pIJ101 (Kieser et al., 1982, Mol Gen. Genet. 185: 223-238) and can be obtained from the American Type Culture Collection, Rockville, MD 20852, under the accession number ATCC 39155. A restriction site and function map of plasmid pIJ702 is presented in Figure 1 of the accompanying drawings.

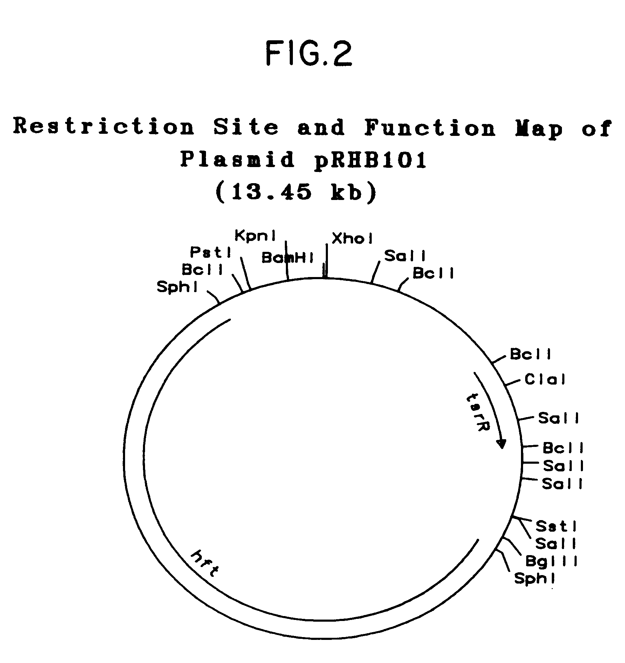

The construction of plasmid pRHB101 from the ~7.8 kb SphI restriction fragment of phage FP43 and SphI-digested plasmid pIJ702 is described in Example 2, below. A restriction site and function map of plasmid pRHB101 is presented in Figure 2 of the accompanying drawings.

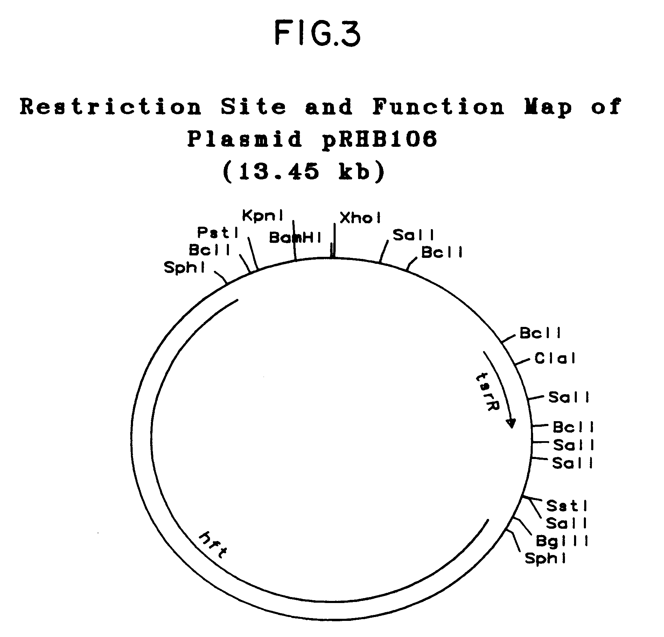

Another illustrative plasmid of the invention was constructed by inserting the ~7.8 kb SphI restriction fragment of phage FP43 into SphI-digested plasmid pMT660 to yield plasmid pRHB106. Plasmid pMT660 is a mutant of plasmid pIJ702 that is temperature-sensitive for replication in Streptomyces lividans (see Birch and Cullum, 1985, J. Gen. Microbiol. 131: 1299-1303). A restriction site and function map of plasmid pRHB106 is presented in Figure 3 of the accompanying drawings. Plasmid pRHB106 can be obtained from S. griseofuscus C581/pRHB106 in substantial accordance with the procedure of Example 3. S. griseofuscus C581/pRHB106 can be obtained from the Northern Regional Research Center under the accession number NRRL 18183.

The above-described illustrative vectors comprise the Streptomyces replicon derived from plasmid pIJ702. However, the present invention is not limited to the use of any particular replicon, for the transduction system of the present invention can be used with a variety of Streptomyces replicons or replicons derived from other organisms. Table I is an illustrative, but not comprehensive, listing of Streptomyces plasmids from which Streptomyces replicons can be obtained. Those skilled in the art recognize that, so long as the replicon function is not disrupted, all or part of the plasmids can be used to construct vectors that contain the hft sequence of the present invention. The plasmid-containing host and depository accession number are also listed in Table 1.

## Table 1

### Streptomyces Plasmids

| Plasmid | Host | Accession Number |
|---|---|---|
| SCP2 | Streptomyces coelicolor A3(2) | NRRL 15042 |
| SCP2* | Streptomyces coelicolor M110 | NRRL 15041 |
| pEL7 | Streptomyces ambofaciens/pEL7 | NRRL 12523 |
| pUC6 | Streptomyces espinosus | NRRL 11439 |
| pUC3 | Streptomyces 3022A | NRRL 11441 |
| SLP1 | Streptomyces lividans | NCIB* 11417 |
| pNM100 | Streptomyces virginiae | NRRL 15156 |
| pEL103 | Streptomyces granuloruber A399 12.13/pEL103 | NRRL 12549 |
| pIJ702 | Streptomyces lividans | ATCC** 39155 |

---

*National Collection of Industrial Bacteria (NCIB),
   Torry Research Station, Post Office Box 31,
   135 Abbey Road, Aberdeen AB98DG, Scotland,
   United Kingdom.
**American Type Culture Collection, Rockville,
   MD  20852.

Restriction fragments used to construct vectors illustrative of the present invention can be conventionally modified to facilitate ligation. For example, molecular linkers can be provided to a particular hft-containing restriction fragment or to DNA comprising vector replication functions. Thus, specific sites for subsequent ligation can be conveniently constructed. In addition, the various hft-containing restriction fragments, origin of replication, or other sequences of a given vector can be modified by adding, eliminating, or substituting certain nucleotides to alter characteristics and to provide a variety of restriction sites for ligation of DNA. Those skilled in the art understand nucleotide chemistry and the genetic code and thus which nucleotides are interchangeable and which DNA modifications are desirable for a specific purpose. It is also noteworthy that a given hft-containing restriction fragment is not limited to a particular position on a cloning vector, as long as critical, vector-controlled functions are not disrupted. Those skilled in the art understand or can readily determine which sites on a vector are advantageous for the ligation or insertion of a particular hft-containing restriction fragment.

Nor is the present invention limited to the use of the ~7.8 kb SphI restriction fragment of phage FP43 to confer transducibility to a particular vector. The ~7.8 kb SphI restriction fragment of phage FP43 is believed to comprise an origin of replication that causes a plasmid containing the origin to replicate by a rolling-circle mechanism, similar to the T4 system in E. coli (see Kreuger et al., 1985, Proc. Natl. Acad. Sci. USA 82: 3345-3349). Linear concatemers of a plasmid containing the FP43 origin of replication, when exposed to intracellular phage FP43 gene products, are packaged by a headful mechanism; and resulting "packages" or particles can inject DNA into cells, and the DNA is recircularized in the transductants.

Because the origin of replication of phage FP43 is smaller than ~7.8 kb, the entire ~7.8 kb SphI restriction fragment of phage FP43 is not required to confer transducibility. Other restriction fragments of phage FP43 that comprise the origin of replication of phage FP43 can be used in place of the ~7.8 kb SphI restriction fragment for purposes of the present transduction system. Of course, larger restriction fragments of phage FP43 that comprise the ~7.8 kb SphI restriction fragment of phage FP43 can also be used for purposes of the present transduction system.

To determine if a particular restriction fragment of phage FP43 contains the FP43 origin of replication, one need only follow the procedure outlined below, substituting the SphI digestion of phage FP43 with some other method (i.e., EcoRI digestion, mechanical shearing, nuclease treatment, etc.) for generating fragments of FP43 DNA. To identify the hft-containing SphI restriction fragment of phage FP43, phage FP43 DNA was digested with restriction endonuclease SphI, and the DNA fragments were cloned into the SphI site located in the mel gene of both plasmids pIJ702 and pMT660.

Inactivation of the mel gene occurs when large DNA fragments are inserted into the SphI site of either plasmid pIJ702 or pMT660. This inactivation is readily detectable, for the mel gene product converts tyrosine into a dark-colored compound. Transformants that contain an intact mel gene thus appear much darker than transformants that do not contain an intact mel gene on tyrosine-containing plates.

The recombinant plasmids resulting from the insertion of phage FP43 DNA into the SphI site of plasmids pIJ702 and pMT660 were transformed into Streptomyces lividans, and thiostrepton-resistant, white transformants were isolated. Plasmid DNA was isolated from the transformants to confirm the presence of DNA inserts. Plasmids containing inserts of FP43 DNA were transformed into S. griseofuscus and S. ambofaciens, and FP43 lysates were prepared on the transformants. The lysates were used to transduce plasmid into wild-type S. griseofuscus and S. ambofaciens, and thiostrepton-resistant transductants were scored. The ~7.8 kb SphI restriction fragment of FP43 (designated hft for high frequency transduction) caused at least a $10^5$-fold increase, as compared with plasmid pIJ702 with no insert, in transduction in S. griseofuscus, as demonstrated in Table 2, below.

## Table 2

### Effects of FP43 DNA inserts on transduction of plasmids pIJ702 and pMT660

| Plasmid | Original Vector | Insert size (kb) | Transduction frequency[a] | |
| --- | --- | --- | --- | --- |
| | | | S. ambofaciens | S. griseofuscus |
| pRHB101 | pIJ702 | 7.8 | | $4.0 \times 10^{-4}$ |
| pRHB102 | pIJ702 | 6.9 | $1.7 \times 10^{-6}$ | |
| pRHB103 | pIJ702 | 2.4 | | $<1.4 \times 10^{-8}$ |
| pRHB104 | pIJ702 | 1.4 | $3.1 \times 10^{-6}$ | |
| pRHB105 | pIJ702 | 2.9 | $<5.0 \times 10^{-7}$ | |
| pRHB106 | pMT660 | 7.8 | | $4.0 \times 10^{-4}$ |
| pRHB107 | pMT660 | 1.5 | | $<4.5 \times 10^{-9}$ |
| pRHB108 | pMT660 | 0.9 | | $<8.3 \times 10^{-7}$ |
| pRHB109 | pMT660 | 1.0 | | |
| pRHB110 | pMT660 | 4.1 | | $<7.7 \times 10^{-6}$ |
| pIJ702 | | - | | $<2.2 \times 10^{-8}$ |
| pMT660 | | - | | $<2.2 \times 10^{-9}$ |

[a] The ratio of the number of transductants to the number of PFU determined on S. griseofuscus.

The average transduction frequency obtained was about $10^{-4}$ per plaque-forming unit. No transduction ($<2.2 \times 10^{-9}$ per PFU) was observed with plasmid pIJ702 containing no inserts. As indicated in Table 2, other plasmids containing inserts of FP43 DNA gave transduction frequencies only slightly higher than observed for plasmid pIJ702. Thus, a generalized enhancement of transduction by cloning random fragments of FP43 was not observed, so this system differs from the Ø105 and SP02 transduction systems studied in Bacillus and the P22 system studied in Salmonella.

The transduction system of the present invention also differs from other systems, because the hft segment can be transduced into most species of Streptomyces. Lysates of phage FP43 prepared on S. griseofuscus containing plasmid pRHB101 and S. griseofuscus containing plasmid pRHB106 were used to transduce many other species of Streptomyces, including both species that do and do not support plaque-formation by FP43. Of the species tested that do not support plaque-formation, some express restriction endonucleases that cleave FP43 DNA (see Cox and Baltz, 1984 J. Bacteriol. 159: 499-504). Of the 13 species tested that support plaque-formation by FP43, only one, Streptomyces lavendulae, was not transduced to thiostrepton resistance by FP43. The results of these transductions are presented in Table 3.

## Table 3

### Interspecies transduction of plasmid pRHB101 by bacteriophage FP43 in Streptomyces

| Strain | Plaque formation | Transduction | [a]Transduction Frequency |
|---|---|---|---|
| S. albus P | + | + | $2.8 \times 10^{-6}$ |
| S. albus J1074 | + | + | $6.1 \times 10^{-8}$ |
| S. ambofaciens | + | + | $4.5 \times 10^{-5}$ |
| S. aureofaciens | + | + | $1.3 \times 10^{-7}$ |
| S. cinnamonensis | + | + | $7.3 \times 10^{-6}$ |
| S. fradiae PM73 | + | + | $1.8 \times 10^{-7}$ |
| S. fradiae M1 | + | + | |
| S. griseofuscus | + | + | $9.6 \times 10^{-4}$ |
| S. griseus | + | + | $3.2 \times 10^{-7}$ |
| S. macrosporeus | + | + | $1.1 \times 10^{-6}$ |
| S. parvulus | + | + | $8.7 \times 10^{-7}$ |
| S. tubercidicus | + | + | $2.3 \times 10^{-7}$ |
| S. tennebrarius E | + | + | $1.2 \times 10^{-4}$ |
| S. lavendulae | + | - | $<2.9 \times 10^{-9}$ |
| S. coelicolor | - | + | $1.9 \times 10^{-5}$ |
| S. felleus | - | + | $5.7 \times 10^{-6}$ |
| S. lividans | - | + | $3.5 \times 10^{-5}$ |
| S. phaeochromogenes | - | + | $8.5 \times 10^{-9}$ |
| S. thermotolerans | - | + | $5.0 \times 10^{-6}$ |
| S. venezuelae | - | + | $1.0 \times 10^{-5}$ |
| S. cirratus | - | + | $2.0 \times 10^{-7}$ |
| S. acromogenes | - | - | $<4.3 \times 10^{-9}$ |
| S. albus G | - | - | $<1.3 \times 10^{-10}$ |
| S. fungicidicus | - | - | $<2.5 \times 10^{-8}$ |
| S. narbonensis | - | - | $<2.5 \times 10^{-8}$ |

[a] The ratio of the number of transductants to the number of PFU determined on S. griseofuscus.

The species transduced included S albus P, which produces SalPI, and isoschizomer of PstI. Plasmids pRHB101 and pRHB106 contain one site for PstI, so the present system provides a means of overcoming such restriction systems. Table 3 also shows that the transduction system of the present invention can even be used to transform organisms resistant to phage FP43 infection. Of the 11 Streptomyces species that do not support plaque-formation by FP43, seven can be transduced to thiostrepton resistance by FP43. Therefore, FP43 clearly attaches and injects DNA into these species, as predicted from the host range analysis (see Cox and Baltz, 1984, J. Bacteriol. 159: 499-504). Phage FP43 is probably restricted to a much greater extent in these species than plasmids pRHB101 and pRHB106. For instance, FP43 has many sites for SphI produced by S. phaeochromogenes and is completely restricted, whereas plasmid pRHB101 has only two sites for SphI and transduces at low but detectable frequency of $8.5 \times 10^{-9}$ per plaque-forming unit.

FP43, like other Streptomyces phages (Chater, K., 1986, Streptomyces phages and their applications to Streptomyces genetics, p. 119-158. In S. W. Queener and L. E. Day (eds), The Bacteria Vol. IX, Antibiotic-producing Streptomyces. Academic Press, New York.) has broad host specificity. Baltz and Cox, 1984, J. Bacteriol. 159: 499-504, demonstrated that phage FP43 formed plaques on 14 of 30 species tested, and the data suggested that the lack of plaque-formation on the 16 species was due primarily to host restriction endonuclease systems. Phage FP43 does not form plaques on S. albus G, S. acromogenes, or S. phaeochromogenes, the producers of restriction endonucleases SalI, SacI, and SphI, respectively; FP43 DNA has many sites for all three enzymes. FP43 forms plaques on S. albus P and S. tubercidicus, the producers of SalPI (PstI) and StuI, respectively; FP43 DNA has no PstI or StuI sites. These observations suggest that FP43 might attach to and inject DNA into most, if not all, species of Streptomyces. The absence of plaque formation by phage FP43 on some Streptomyces species represents host restriction rather than a failure of phage FP43 to attach to and inject DNA into the host cells. The present transduction system provides a convenient means for avoiding such restriction systems.

If FP43 packaged a plasmid containing a relatively small number of restriction sites, it might transduce that plasmid into a strain that is highly restricting for FP43. Of the strains listed in Table 3, Streptomyces griseofuscus, S. ambofaciens, S. lividans, S. parvulus, and S. albus J1074 are relatively non-restricting; S. albus G (SalI), S. lavendulae (SlaI), S. phaeochromogenes (SphI), S. acromogenes (SacI, SacII, and S. tubercidicus (StuI) produce well characterized restriction systems (shown in parenthetical remark following the strain name); and strains S. aureofaciens, S. cirratus, S. coelicolor, S. griseus, S. narbonensis, S. thermotolerans, S. venezuelae, and S. macrosporeus are suspected of producing restriction systems. The transduction system of the invention works well in 13 of the 14 strains tested that FP43 forms plaques on and works well in 7 of 11 strains that FP43 does not form plaques on. Four of the five strains that were not transduced produce potent restriction systems; S. albus G produces SalI, an enzyme that cuts plasmid pRHB101 five times. Only about 20% of the strains tested produce restriction systems that are not readily bypassed.

Two of the species transduced produce well-characterized enzymes that produce restriction endonucleases that cut the transducing plasmid. S. albus P produces SalPI, and isoschizomer of PstI that cuts plasmid pRHB101 one time, and S. phaeochromogenes produces SphI, which cuts plasmid pRHB101 two times. Phage FP43 transduced S. albus P and S. phaeochromogenes at frequencies about $10^2$-fold and $10^4$-fold lower than those obtained on the nonrestricting S. griseofuscus. In the case of S. albus P, the relative transduction frequency was improved 100-fold by preparing the transducing lysate in S. albus P. It appears, therefore, that the plasmid was efficiently modified for the SalPI restriction system after replication in S. albus P. This procedure, often referred to as "laundering" the DNA, can be used in other species to improve transduction efficiencies and is believed to work by a mechanism involving modification (i.e., methylation) of the transducing DNA by the "laundering" organism (S. albus P in the procedure above).

This laundering procedure was carried out by transducing S. albus P to thiostrepton resistance using an FP43 lysate prepared on S. griseofuscus containing plasmid pRHB101. A subsequent FP43 lysate was prepared on S. albus P containing plasmid pRHB101. The two transducing lysates were compared for their relative abilities to transduce S. griseofuscus and S. albus P strains not containing plasmid. Table 4, below, shows that the lysate prepared on S. griseofuscus transduced S. albus P to thiostrepton resistance about 15% as efficiently as it transduced native S. griseofuscus. The lysate prepared on S. albus P, however, transduced both species at about equal efficiencies. Thus passageing the plasmid through the restricting host increased the relative transduction on the restricting host by about 100-fold.

8

**Table 4**

Transduction of Streptomyces griseofuscus and S. albus P host with FP43 lysates prepared on S. griseofuscus/pRHB101 or S. albus P/pRHB101

| Source of transducing lysate | Transduction frequency on S. griseofuscus | S. albus P | Relative Transduction (S. albus/S. griseofuscus) |
|---|---|---|---|
| S. griseofuscus | $6.6 \times 10^{-4}$ | $1.0 \times 10^{-5}$ | 0.015 |
| S. albus P | $2.4 \times 10^{-5}$ | $3.5 \times 10^{-5}$ | 1.5 |

The very high frequency of successful transductions obtained in Streptomyces species that do not support FP43 plaque-formation suggested that the lack of plaque-formation by Streptomyces phages in other actinomycete genera is due to events beyond phage attachment and injection and that the FP43 transduction system of the present invention could be used to transduce other actinomycete genera. Intergeneric transductions can also be facilitated by using plasmids with very broad host ranges; plasmid pIJ702 and derivatives replicate not only in Streptomyces but also in Saccharopolyspora, Amycolatopsis, and Thermomonospora species.

Lysates of phage FP43 prepared on S. griseofuscus/pRHB101 were mixed with cells prepared from several species of different actinomycete genera, and plaque-formation and transduction was scored. The results are presented in Table 5, below.

## Table 5

### Intergeneric transduction of plasmid pRHB101 by bacteriophage FP43

| Strain | Plaque formation | Transduction | [a]Transduction Frequency |
|---|---|---|---|
| Chainia minutisclerotica ATCC 19346 | + | + | $1.1 \times 10^{-6}$ |
| Chainia ochracea ATCC 15814 | - | + | $6.1 \times 10^{-7}$ |
| Chainia olivacea ATCC 15722 | - | + | $4.1 \times 10^{-8}$ |
| Saccharopolyspora erythraea ATCC 11635 | - | + | $6.0 \times 10^{-7}$ |
| Saccharopolyspora hirsuta ATCC 27875 | - | + | $1.4 \times 10^{-8}$ |
| Streptoverticillium oliverticulli ATCC 23943 | + | + | $7.1 \times 10^{-9}$ |
| Streptoverticillium kentuckense NRRL B-1831 | - | + | $2.1 \times 10^{-7}$ |
| Streptoverticillium albireticuli NRRL B-1670 | - | - | $<7.1 \times 10^{-10}$ |

[a] The ratio of the number of transductants to the number of PFU determined on S. griseofuscus.

FP43 caused plaque-formation on only Streptoverticillium oliverticulli and Chainia minutisclerotica. However, transduction was successful not only in Streptoverticillium and Chainia, but also in Saccharopolyspora. These results indicate that FP43 can attach and inject plasmid DNA into a variety of actinomycete genera and that plasmid pRHB101 can establish and replicate in many different genera. Thus, the transduction system described here provides a very powerful technique to move cloned genes between a variety of Streptomyces species and into at least several other actinomycete genera and eliminates the need to develop transformation systems for each species of interest. This should accelerate the applications of recombinant DNA technology in actinomycetes to produce novel or hybrid antibiotics.

The wide host range of the transduction vectors of the present invention provides an enormous advantage over protoplast-transformation in experiments and procedures for transferring genes from one organism to another. Because this transduction system works in such a diversity of organisms, the optimal conditions for transducing one organism may differ from the optimal conditions for transforming a different organism. One condition that can be optimized is the multiplicity of infection (m.o.i.).

To understand how m.o.i. and transduction efficiency are related, it is important to understand transduction. To make a transducible plasmid, the hft sequence of phage FP43 is incorporated into a recombinant DNA expression vector. That vector (i.e., plasmid pRHB101 or plasmid pRHB106) is then transformed, by conventional protoplast-transformation procedures, into an organism, such as Streptomyces griseofuscus C581 (ATCC 23916). The resulting transformants are then infected with phage FP43. Upon phage infection, plasmids containing the hft sequence are replicated and packaged into phage heads. The resulting lysate contains noninfective particles that have packaged the hft-containing plasmid DNA.

The resulting lysate also contains, however, infective phage particles that have packaged the phage FP43 genome. These "wild-type" infective particles can cause lysis, and are thus referred to as "plaque-forming units" or "PFU", of the recipient host cells when the lysate is used in a subsequent transduction.

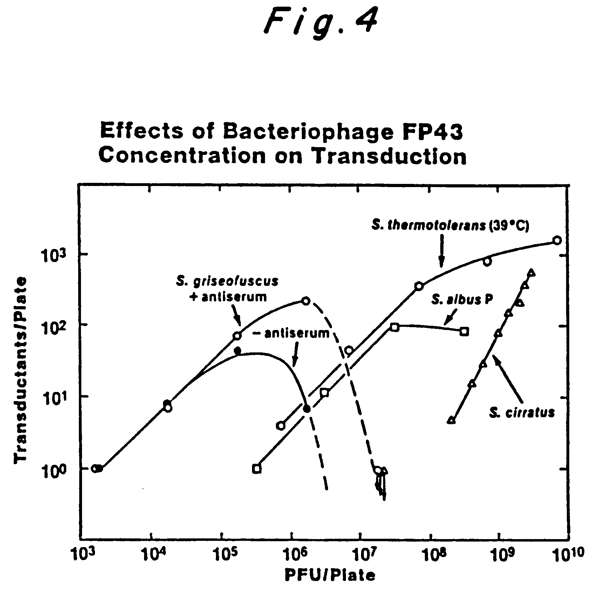

Figure 4 shows several typical responses in transduction frequency to increased phage concentration for species that are hosts for FP43 and for species that are not. While the efficiencies of transduction observed in different species of Streptomyces vary from about $10^{-8}$ to $10^{-4}$ per PFU, transduction efficiency has little bearing on the maximum number of transductants obtainable on a transduction plate. The highest transduction frequencies are obtained on nonrestricting or marginally restricting hosts that are susceptible to lysis by FP43.

In Streptomyces griseofuscus, a nonrestricting host on which the transducing lysates were prepared, the frequency of transductants increased linearly with increasing plaque-forming units between about $10^3$ and $10^5$ PFU per plate and then declined if anti-FP43 antiserum was not present. The procedure for preparing anti-FP43 antiserum is described in Example 6. When antiserum was added at 4 hours after the addition of phage, the frequency of transduction increased linearly to about $10^6$ PFU per plate and then declined. Thus the addition of antiserum is useful in minimizing lysis of transductants in a limited range of phage multiplicity. Furthermore, little or no lysis, even in the absence of phage antiserum, is observed when the strain to be transduced is a lysogen for the transducing phage. Substantial lysis of transductants is observed at higher phage concentrations. Thus at $2 \times 10^6$ PFU per plate, 200 transductants were obtained per plate.

However, with Streptomyces thermotolerans, which is highly restricting for and does not support plaque formation of FP43, as many at $10^{10}$ PFU could be added per plate without lysing the transductants. With S. thermotolerans the frequency of transductants increased proportionally with increasing PFU up to about $10^8$ PFU per plate, then continued to rise at a slope of less than one up to nearly $10^{10}$ PFU per plate. Thus, there was no problem with lysis of transductants at high phage multiplicities. In this case, ~1500 transductants were observed from about $10^{10}$ PFU. Because the numbers of phage particles and cells added in the typical experiments are not serious limitations, very high multiplicities of phages and high cell densities can be used for highly restricting strains. These are clear advantages over protoplast systems where transformation is often most efficient at low protoplast concentrations, where uptake of plasmid is relatively inefficient, and where protoplast regeneration seldom approaches 100%.

FP43 forms plaques on Streptomyces albus P at an efficiency-of-plating (EOP) of about 10% relative to the maximum EOP observed on S. griseofuscus. The frequency of transductants of S. albus P increased linearly with increasing PFU to about $3 \times 10^7$ PFU per plate and then plateaued. However, the relative efficiency of transduction per PFU was about 100-fold lower than that observed on S. griseofuscus.

With Streptomyces cirratus, a strain that does not support plaque-formation by FP43, a very different response was observed. With S. cirratus, no transduction was observed at phage concentrations less than $10^8$ PFU per plate, but above this concentration the frequency of transductants increase proportionally with the square of phage concentration. As with S. thermotolerans there was no problem with lysis on the plate, but in fact there appeared to be a synergistic effect at high phage multiplicities.

This apparent bimolecular interaction suggests that transduction requires either two plasmids or one plasmid and one phage genome to establish plasmid replication. One possibility is that coinfection of plasmid and phage DNA reduces the effects of restriction. Perhaps the phage DNA initiates an abortive infection that competes out restriction endonuclease, thus allowing more efficient initiation of plasmid replication, although other possibilities could explain this interesting phenomenon. In any event, many transductants can be obtained at high phage multiplicity in a restricting background when bimolecular transduction kinetics are encountered.

The present invention provides a variety of means for overcoming a host cell's endogenous DNA restriction/modification system. Elevated temperature can often inhibit secondary metabolic functions such as antibiotic production and sporulation in Streptomyces. Many restriction/modification systems may be regulated in a similar way, so growth of cells at elevated temperature might result in decreased expression of restriction and increased transduction in some restricting strains. To demonstrate this method of overcoming endogenous restriction systems, S. griseofuscus, S. albus P, S. phaeochromogenes, S.

thermotolerans, and S. kentuckense were grown at 29°C and 39°C before transduction and incubated at 29°, 34° or 42°C after transduction. Table 6 below details the results of this experiment.

## Table 6

### Effects of temperature on transduction of plasmid pRHB101 by FP43

| Strain | Temperature (°C) | | Transduction | [a]Relative |
|--------|--------|--------------|--------------|-------------|
| | Growth | Transduction | frequency | transduction |
| S. griseofuscus | 29 | 29 | $3.3 \times 10^{-4}$ | 1.1 |
| | 29 | 34 | $2.9 \times 10^{-4}$ | 1.0 |
| | 29 | 42 | $1.6 \times 10^{-4}$ | 0.55 |
| | 39 | 29 | $5.0 \times 10^{-5}$ | 0.17 |
| | 39 | 34 | $3.0 \times 10^{-5}$ | 0.10 |
| | 39 | 42 | $7.3 \times 10^{-5}$ | 0.25 |
| S. albus P | 29 | 29 | $6.2 \times 10^{-6}$ | 1.05 |
| | 29 | 34 | $5.9 \times 10^{-6}$ | 1.0 |
| | 29 | 42 | $2.7 \times 10^{-6}$ | 0.46 |
| | 39 | 29 | $4.3 \times 10^{-6}$ | 0.73 |
| | 39 | 34 | $4.3 \times 10^{-6}$ | 0.73 |
| | 39 | 42 | $3.9 \times 10^{-6}$ | 0.66 |
| S. phaeochromogenes | 29 | 29 | $5.0 \times 10^{-9}$ | 1.0 |
| | 29 | 34 | $5.0 \times 10^{-9}$ | 1.0 |
| | 29 | 42 | $<5.0 \times 10^{-9}$ | $<1.0$ |
| | 39 | 29 | $3.0 \times 10^{-8}$ | 6.0 |
| | 39 | 34 | $5.0 \times 10^{-8}$ | 10.0 |
| | 39 | 42 | $2.0 \times 10^{-8}$ | 4.0 |
| S. thermotolerans | 29 | 34 | $1.5 \times 10^{-8}$ | 1.0 |
| | 39 | 34 | $5.2 \times 10^{-6}$ | 350.0 |
| S. kentuckense | 29 | 34 | $4.7 \times 10^{-9}$ | 1.0 |
| | 39 | 34 | $2.2 \times 10^{-7}$ | 47.0 |

[a] Transduction frequencies were normalized to those obtained when cells were grown at 29°C and transductions were carried out at 34°C.

In the nonrestricting S. griseofuscus, transduction was decreased 5 to 10-fold relative to the 29°C control by incubating cells at 39°C, whereas S. albus P showed very little variation in transduction frequencies with variation in temperature for cell growth or transduction. However, S. phaeochromogenes cells grown at 39°C were 4 to 10-fold more transducible than cells grown at 29°C. These results suggest that the SphI restriction system functions poorly at elevated temperature. Maximum transduction was obtained when the cultures were grown at 39°C and the transduction plates were incubated at 34°C. S. thermotolerans and S. kentuckense cells grown at 39°C were 350-fold and 47-fold more transducible, respectively, than the corresponding cells grown at 29°C. Thus the temperature for cell growth can have a marked influence on efficiency of transduction in some species.

In any event, the data demonstrates that this transduction system can be readily manipulated by changing cell growth parameters to optimize transduction for particular species. This differs from the protoplast transformation systems that often have very inflexible requirements for cell growth before protoplast formation in order to obtain efficient regeneration of cells. For instance, growth of cells at elevated temperature can cause drastic inhibition of cell regeneration from protoplasts.

The recombinant DNA cloning vectors of the present invention have broad utility and help fill the need for suitable cloning vehicles for use in Streptomyces and related organisms. Moreover, the ability of the present vectors to confer antibiotic resistance provides a functional means for selecting transductants. This is important because of the practical necessity for determining and selecting the particular cells that have acquired vector DNA in a transformation procedure.

Additional DNA segments that lack functional tests for their presence can also be inserted into the present vectors, and transductants containing the nonselectable DNA can be isolated by selection for carbomycin resistance. Such non-selectable DNA segments can be inserted at any site, except within regions necessary for transduction or within the antibiotic resistance-conferring gene used for selection, and include, but are not limited to, genes that specify antibiotic modification enzymes and regulatory genes of all types.

The vectors of the present invention are also useful for ensuring that linked DNA segments are stably maintained in host cells over many generations. These genes or DNA fragments are maintained by exposing the transductants to selective pressure based upon the markers (i.e., an antibiotic resistance-conferring gene) present on the vector. Therefore, transductants that lose the vector cannot grow and are eliminated from the culture. Thus, the vectors of the present invention can stabilize and maintain DNA sequences of interest.

The cloning vectors and transductants of the present invention provide for the cloning of genes to improve yields of various products that are currently produced in Streptomyces and related cells. Examples of such products include, but are not limited to, Streptomycin, Tylosin, Cephalosporins, Actaplanin, Narasin, Monensin, Tobramycin, Erythromycin, and the like. The present invention also provides selectable vectors that are useful for cloning, characterizing, and reconstructing DNA sequences that code for: commercially important proteins such as, for example, human insulin, human proinsulin, glucagon, interferon and the like; enzymatic functions in metabolic pathways leading to commercially important processes and compounds; or control elements that improve gene expression. These desired DNA sequences also include, but are not limited to, DNA that codes for enzymes that catalyze synthesis of derivatized antibiotics such as, for example, Streptomycin, Cephalosporin, Tylosin, Actaplanin, Narasin, Monensin and Erythromycin derivatives, or for enzymes that mediate and increase bioproduction of antibiotics or other products. The capability for isolating and using such DNA segments allows for increasing the yield and availability of antibiotics that are produced by Streptomyces and related organisms.

Streptomyces can be cultured in a number of ways using any of several different media. Preferred carbohydrate sources in a culture medium include, for example, molasses, glucose, dextrin, and glycerol. Nitrogen sources include, for example, soy flour, amino acid mixtures, and peptones. Nutrient inorganic salts are also incorporated and include the customary salts capable of yielding sodium, potassium, ammonium, calcium, phosphate, chloride, sulfate, and like ions. As is necessary for the growth and development of other micro-organisms, essential trace elements are also added. Such trace elements are commonly supplied as impurities incidental to the addition of other constituents of the medium.

Streptomyces is grown under aerobic culture conditions over a relatively wide pH range of about 5 to 9 at temperatures ranging from about 15° to 40°C. For plasmid stability and maintenance, it is desirable to start with a culture medium at a pH of about 7.2 and maintain a culture temperature of about 30°C.

The following examples further illustrate and describe the invention disclosed herein. The invention is not limited in scope by reason of any of the following Examples; sources of reagents or equipment are provided merely for convenience and in no way limit the invention. Both an explanation of and the actual procedures for constructing the invention are described where appropriate.

Example 1

## Isolation of Phage FP43 from *Streptomyces griseofuscus* C581 (FP43)

A. List of Solutions

The following solutions are referred to throughout the Examples and are presented here for clarity.
1. P Media (~100 ml):

| Ingredient | Amount |
|---|---|
| Sucrose | 10.3 g |
| $K_2SO_4$ | 0.025 g |
| Trace element solution (see #3) | 0.2 ml |
| $MgCl_2 \cdot 6H_2O$ | 0.203 g |
| Water | 80 ml |

After autoclaving add:

| | |
|---|---|
| $KH_2PO_4$ (0.5%) | 1 ml |
| $CaCl_2 \cdot 2H_2O$ (3.68%) | 10 ml |
| (N-tris-(hydroxymethyl)-methyl-2-aminoethane sulphonic acid), "TES" buffer, 0.25 M, pH=7.2 | 10 ml |

2. Trace element solution (~1 L):

| Ingredient | Amount |
|---|---|
| $ZnCl_2$ | 40 mg |
| $FeCl_3 \cdot 6H_2O$ | 200 mg |
| $CuCl_2 \cdot 2H_2O$ | 10 mg |
| $MnCl_2 \cdot 4H_2O$ | 10 mg |
| $Na_2B_4O_7 \cdot 10H_2O$ | 10 mg |
| $(NH_4)_6Mo_7O_{24} \cdot 4H_2O$ | 10 mg |
| $H_2O$ | 1 L |

3. R2 Regeneration Media, also known as Modified R2 Media (~1 L):

| Ingredient | Amount |
|---|---|
| Sucrose | 103 g |
| $K_2SO_4$ | 0.25 g |
| Trace element solution | 2 ml |
| $MgCl_2 \cdot 6H_2O$ | 10.12 g |
| glucose | 10 g |
| L-asparagine $\cdot 1H_2O$ | 2.0 g |
| casamino acids | 0.1 g |
| Agar | 22 g |
| Water | to 700 ml |

The pH is adjusted to pH = 7.2 before autoclaving.
After autoclaving, add:

| | |
|---|---|
| $KH_2PO_4$ (0.05 g/100 ml) | 100 ml |
| $CaCl_2$ (2.22 g/100 ml) | 100 ml |
| TES Buffer (5.73 g/100 ml, pH = 7.2) | 100 ml |

4. Soft Nutrient Agar (SNA, ~1 L):

| Ingredient | Amount |
|---|---|
| Difco Nutrient Broth | 8 g |
| Agar | 5 g |

5. R2YE medium is R2 medium with 20 ml of 25% yeast extract added per liter.
6. Yeast Extract - Malt Extract (YEME, ~1 L):

| Ingredient | Amount |
|---|---|
| Yeast extract | 3 g |
| Peptone | 5 g |
| Malt extract | 3 g |
| Glucose | 10 g |

7. YEME + 34% Sucrose Liquid Complete Media is YEME with 340 g/L of sucrose.

8. YMX Media (~1 L):

| Ingredient | Amount |
|---|---|
| Yeast extract | 3 g |
| Malt extract | 3 g |
| Glucose | 2 g |
| Agar | 20 g |

9. YMX Agar is 0.3% yeast extract, 0.3% malt extract, 0.2% dextrose, and 2.0% agar.

10. CSI Media (~1 L):

| Ingredient | Amount |
|---|---|
| Soybean meal | 15 g |
| Casein | 1 g |
| Cerelose | 25 g |
| Blackstrap molasses | 3 g |
| $CaCO_3$ | 2.5 g |
| Czapek Mineral Stock | 2 ml |
| Water (deionized) | 1 L |
| pH adjusted to 7.2 prior to sterilization | |

11. Czapek's Mineral Mix (~1 L):

| | |
|---|---|
| KCl | 100 g |
| $MgSO_4 \cdot 7H_2O$ | 100 g |
| Deionized Water | 900 ml |

$FeSO_4 \cdot 7H_2O$ (2 g) was dissolved in 100 ml deionized water containing 2 ml of concentrated HCl. This solution was added to the above $KCl/MgSO_4 \cdot 7H_2O$ solution to complete preparation of the Czapek's Mineral Mix.

12. Bennett's Agar (~1 L):

| Ingredient | Amount |
|---|---|
| Deionized $H_2O$ | 1000 ml |
| Potato Dextrin | 10 g |
| N-Z Amine A | 2 g |
| *Gibco bactoagar | 15 g |
| Gibco beef extract | 2 g |
| Yeast extract | 1 g |
| Czapek's mineral stock | 2 ml |

*Gibco Laboratories, 3175 Staley Road, Grand Island, N.Y.  14072

13. NC Broth

NC broth contains 8 g of Difco (P.O. Box 1058, Detroit, MI 48232) nutrient broth per liter of deionized $H_2O$ and is also 4 mM in $Ca(NO_3)_2$.

14. TES Buffer

TES is an abbreviation for 2-{(tris-[hydroxymethyl]-methyl)amino}ethanesulfonic; to prepare TES buffer, a 1 M solution of TES acid (125.6 g/500 ml) is mixed with an equal volume of 1 M TES base and then diluted with distilled water to 0.25 M in TES.

15. Sevag

Sevag is a 24:1 mixture of chloroform:isoamyl alcohol

16. Ø Buffer Ø buffer is 10 mM TES and 10 mM $Ca(NO_3)_2$.

17. TE Buffer

TE buffer contains 10 mM Tris-HCl, pH = 8, and 1 mM $Na_2EDTA$.

18. R2 Overlays (per 1 L)

| Ingredient | Amount |
|---|---|
| Sucrose | 103 g |
| $MgCl_2$ | 10.12 g |
| 0.151 M $CaCl_2$ | 100 ml |
| TES Buffer | 100 ml |
| Gibco Agar | 4.1 g |
| Distilled $H_2O$ | to 1 L |

19. TSS Broth

TSS broth is prepared by adding 51.9 ml of 60% sucrose to 250 ml of TSB. As used herein, the TSS broth also contains enough glycine to give a final glycine concentration of 0.5%.

B. Phage Isolation

Phage lysates and DNA were prepared in substantial accordance with the procedure described in Cox and Baltz, 1984, J. Bacteriol. 159: 499-504. A lyophilized culture of Streptomyces griseofuscus C581(FP43) is obtained from the Northern Regional Research Center (NRRL), Agricultural Research Service, Peoria, IL 61604 under the accession number NRRL 18184. The lyophilized culture is used to inoculate 10 ml of NC broth; the culture is then incubated at 29°C in a gyrotory incubator overnight (~16 hours). The culture is centrifuged to remove the cells and cellular debris; the supernatant was then passed through a 0.45 µ filter, and the filtrate was saved and contained phage FP43 particles.

A lyophil of Streptomyces griseofuscus C581 is obtained from the American Type Culture Collection (ATCC), Rockville, MD 20852 under the accession number ATCC 23916. The lyophilized culture is used to inoculate 10 ml of TSB broth (Baltimore Biological Laboratories, Inc. (BBL), P.O. Box 243, Cockeysville, MD 21031) in a 50 ml flask. The culture is incubated at 29°C in a gyrotory incubator overnight.

Four 100 µl aliquots of the overnight culture of Streptomyces griseofuscus C581 are prepared and mixed, respectively, with 1.0 ml, 100 µl, 10 µl, and 1 µl of the lysate solution. The mixtures are then individually plated on NC agar (NC broth with 15 g/L agar) in 100 x 15 mm Petri plates and incubated at 34°C overnight. The following morning, the plates are examined, and the plate showing nearly confluent lysis is used to prepare the phage FP43 stock solution. The FP43 stock solution is prepared by adding ~5 ml of NC broth to the plate showing nearly confluent lysis, incubating the plate at room temperature for one to two hours, and collecting the broth from the plate. The solution was centrifuged and the resulting supernatant passed through a 0.45 µ filter to remove debris. The resulting phage FP43 solution typically contains $10^9$ to $10^{10}$ FP43 particles per ml--the exact titer is determined by plating several dilutions of the phage stock on a sensitive strain, such as S. griseofuscus C581.

C. Phage DNA Isolation

To prepare phage FP43 DNA, the procedure described in Example 1B was followed, except the lysates were prepared using four to six 9.5" x 9.5" Petri dishes. The plates were washed with 50 ml of NC broth to collect the phage particles. The lysates were centrifuged and the supernatants passed through a 0.45 µ filter to remove cellular debris. The lysate was then centrifuged for 2 hours at 25°C at 30,000 rpm to pellet the phage particles. Each pellet was resuspended in 1 ml of Ø buffer; this solution was centrifuged in a tabletop centrifuge to pellet material that did not go back into solution. The supernatant was then layered on top of a CsCl gradient composed as follows (from most dense to least dense): 750 µl of 1.7 ρ CsCl in Ø buffer; 750 µl of 1.6 ρ CsCl in Ø buffer; 750 µl of 1.4 ρ CsCl in Ø buffer; and ~2.45 ml of phage FP43 in Ø buffer. The gradient was prepared in a polyallomer tube ($\frac{1}{2}$" x $\frac{1}{2}$"), which was placed in an SW50.1 rotor (Beckman Instruments, Inc., Spinco Division, P.O. Box 10200, Palo Alto, CA 94304). The solution was centrifuged at 25,000 rpm at 15°C for 1 hour and yielded two bands: a brown-tinged top band and a blue-tinged lower band. The lower band was collected with a syringe and dialyzed against 2 to 3 liters of Ø buffer overnight.

The dialyzed band was extracted for 30 minutes with 1.5 volumes of Ø buffer-saturated phenol (25 ml phenol:10 ml Ø buffer) and then re-extracted for 10 minutes with another 1.5 volumes of Ø buffer-saturated phenol. The phage band was then extracted 3 times with one volume of Sevag. The phage DNA was precipitated with 0.1 volume of 3 M sodium acetate (NaOAc), pH = 8.0, and 1 volume of isopropanol. The precipitated phage FP43 DNA was spooled from the solution, washed with 70% ethanol and resuspended in ~1 ml of TE buffer, yielding a solution containing ~0.5 mg/ml of phage FP43 DNA.

Example 2

Construction of Plasmid pRHB101

Plasmid isolations were carried out in substantial accordance with the procedures of Birnbolm and Doly, 1979, Nucleic Acids Res. 7: 1513-1523 and Kieser, 1984, Plasmid 12: 19-36.

A. Isolation of Plasmid pIJ702

A lyophilized culture of Streptomyces lividans/pIJ702 (ATCC 39155) is used to inoculate 10 ml of TSS medium containing 25 µg/ml thiostrepton. The culture is incubated at 29°C until the cells reach early stationary phase. The culture was then homogenized, and 5 ml of the homogenized culture were used to inoculate 100 ml of TSS also containing thiostrepton. The 100 ml of culture were incubated at 29°C until the Streptomyces lividans/pIJ702 cells reached stationary phase.

The cells were collected and washed once with a 10.3% sucrose solution. The cells were then suspended in 24 ml of 20.3% sucrose, and 6 ml of 5X lysozyme solution (125 mM Tris-HCl, pH = 8; 125 mM Na$_2$EDTA, pH = 8; 10 mg/ml lysozyme; and 10.3% sucrose) were added. The solution was mixed and then incubated at 30°C for 30-60 minutes, and then, about 18 ml of a solution that was 0.3 m NaOH, 1% SDS, and prewarmed to 50°C were added, mixed and the resulting mixture incubated at 80°C for 10 minutes. The mixture was then cooled to room temperature, and 12 ml of a solution made by mixing 500 g

EP 0 281 356 B1

phenol, 500 g CHCl$_3$, and 0.5 g 8-hydroxyquinoline in 200 ml H$_2$O were added and mixed well with the cell-extract. The phases were separated by centrifugation at 6000-8000 rpm for 10 minutes; approximately 45 ml of the resulting upper phase were transferred to a clean bottle.

Next, 4.5 ml of 3 M NaOAc and 50 ml of isopropanol were added to the supernatant, and the solution was mixed and left at room temperature for 30 minutes. The solution was then centrifuged (8000 rpm for 30 minutes) and the resulting supernatant discarded. The pellet was resuspended in 7.5 ml TE buffer (10 mM Tris-HCl, pH = 8, and 1 mM EDTA) containing 8 g of CsCl. About 0.5 ml of a 10 mg/ml solution of ethidium bromide was added to the solution, which was then centrifuged at 40,000 rpm for 48 hours at 29°C. The fraction containing the plasmid band was extracted 3-5 times with isopropanol saturated with TE buffer and CsCl to remove the ethidium bromide. After the extractions, the sample was diluted with four volumes of TE buffer, and then, two-and-one-half volumes of ethanol were added. The resulting solution was mixed and incubated overnight at -20°C.

The precipitate resulting from the overnight incubation at -20°C was collected by centrifugation (10,000 rpm for 30 minutes), dried, and reprecipitated twice. The precipitations were done by suspending the pellet in TE buffer, adding NaOAc to 0.3 M, adding 2.5 volumes ethanol, chilling at -70°C for 10-15 minutes, and then centrifuging the solution as above. The procedure yields about 100 µg of plasmid pIJ702 DNA, which was suspended in TE buffer at a concentration of 1 µg/µl and stored at 4°C. A restriction site and function map of plasmid pIJ702 is presented in Figure 1 of the accompanying drawings.

About 3 µg (3 µl) of plasmid pIJ702 DNA were added to 5 µl of 10X SphI buffer (60 mM Tris-HCl, pH = 7.4; 1.5 M NaCl; 60 mM MgCl$_2$; 100 mM 2-mercaptoethanol; and 1 mg/ml bovine serum albumin (BSA)), 37 µl of H$_2$O, and 5 µl (~20 units; unit definitions herein correspond to those of New England Biolabs, 32 Tozer Road, Beverly, MA 01915-9990, unless otherwise indicated) of restriction enzyme SphI. The resulting reaction was incubated at 37°C for one hour. About 5 µl of 10 X kinase buffer (0.1 M MgCl$_2$; 50 mM dithiothreitol (DTT); and 0.5 M Tris-HCl; pH = 9.5), 10 µl of a 1:3 dilution (in kinase buffer) of calf-intestinal alkaline phosphatase (CAP, obtained from Boehringer-Mannheim Biochemicals, 7941 Castleway Drive, P.O. Box 50816, Indianapolis, IN 46250), and 35 µl of H$_2$O were added to the solution of SphI-digested plasmid pIJ702 DNA, and the solution was incubated at 38°C for 30 minutes. The mixture was then placed at 65°C, and another 10 µl of a 1:3 dilution of CAP were added to the solution which was incubated at 65°C for another 30 minutes. Yet another 10 µl of a 1:3 dilution of CAP was again added to the solution, which was incubated at 65°C for another 30 minutes. Then, the SphI-digested, CAP-treated plasmid pIJ702 DNA was extracted twice with Ø buffer-saturated phenol, extracted three times with ether, and collected by adjusting the sodium acetate (NaOAc) concentration of the reaction mixture to 0.30 M, adding two volumes of ethanol, chilling the reaction mixture to -70°C, and centrifuging to pellet the precipitated DNA. The pellet was resuspended in 50 µl of TE buffer.

About 7.5 µg of phage FP43 DNA in ~20 µl of TE buffer were added to 5 µl of 10X SphI buffer, 20 µl of H$_2$O, and 5 µl (~20 units) of restriction enzyme SphI, and the resulting reaction was incubated at 37°C for one hour. The SphI-digested phage FP43 DNA was extracted twice with Ø buffer-saturated phenol, extracted three times with ether, precipitated, and resuspended in 50 µl of TE buffer.

The SphI-digested, alkaline phosphatase-treated plasmid pIJ702 DNA was added to the SphI-digested phage FP43 DNA, 37.5 µl of 10X ligase buffer (660 mM Tris-HCl, pH = 8; 66 mM MgCl$_2$; 200 mM dithiothreitol; 10 mM ATP; and 50 µg/ml BSA), and 219 µl of H$_2$O. About 19 µl of T4 DNA ligase were added to the solution of DNA, and the resulting reaction was incubated at 15°C overnight (~16 hours). The ligated DNA constituted the desired plasmid pRHB101 DNA. A restriction site and function map of plasmid pRHB101 is presented in Figure 2 of the accompanying drawings. The ligated DNA, after precipitation and resuspension in 10 µl of TE buffer, was used to transform Streptomyces lividans TK23 as described in Example 4, below.


Example 3


Isolation of Plasmid pRHB106


Plasmid pRHB106 was constructed in accordance with the foregoing procedure except that plasmid pMT660 was used instead of plasmid pIJ702. However, for convenience, plasmid pRHB106 can also be obtained in Streptomyces griseofuscus C581 from the NRRL under the accession number NRRL 18183.

Plasmid pRHB106 need not be isolated from a phage FP43 genomic library as is described for plasmid pRHB101 in Examples 2 and 4. Instead, S. griseofuscus C581/pRHB106 can be used to prepare a transducing lysate as described in Example 4. The transducing lysate will contain phage particles that have packaged plasmid pRHB106 DNA and can be used in transductions as described in Example 5.

19

Example 4

<div align="center">

## Identification of Plasmid pRHB106
## Illustrating the Isolation of an FP43 hft
## Sequence-Containing Vector

</div>

The procedure set forth below can be used to identify any hft-containing FP43 restriction fragment. In Example 2, a genomic library of phage FP43 was constructed, which included the hft-containing plasmid pRHB101. The procedure set forth below demonstrates how plasmid pRHB101 was isolated from the genomic library. Briefly stated, the procedure first involves protoplast transformation of Streptomyces lividans TK23. These transformants, identified on the basis of their mel⁻, tsrR phenotype, were examined for size of insert (FP43) DNA. A group of plasmids, each containing a different SphI restriction fragment and constituting a genomic library of phage FP43, were isolated and used to transform S. griseofuscus. The S. griseofuscus transformants were infected with phage FP43 to prepare lysates. The lysates were examined for their ability to transduce S. griseofuscus. The lysate that yielded the highest transduction frequency comprised plasmid pRHB101 packaged into phage particles.

<div align="center">

## A.  Transformation of Streptomyces lividans

</div>

Streptomyces lividans TK23 (NRRL 15826) was grown in a 10 ml culture for 40-48 hours at 30°C in TSS broth. The culture was then homogenized and sonicated, and the mycelial fragments were recovered by centrifugation (800Xg for 10 minutes in a bench top centrifuge) and washed once with 10 ml of P media. The mycelial fragments were resuspended in 10 ml of P media containing 5 to 10 mg/ml of egg-white lysozyme (Calbiochem-Behring, P.O. Box 12087, San Diego, CA 92112) and incubated for 1 hour at 4°C. During this interval the suspension was pipetted up and down once or twice to disperse clumps. The protoplasts were recovered by centrifugation (800Xg for 10 minutes) and washed twice with 10 ml of p medium. The protoplasts were then suspended in 10 ml of P medium.

About 200 $\mu$l of protoplasts and about 0.3 to 0.5 $\mu$g of the ligated DNA prepared in Example 2 was added together per transformation. About 0.5 ml of 20% PEG 1000 in P medium was then added to the protoplast-DNA mixture. The mixture was pipetted up and down once or twice to mix the contents. At this point, the suspension was plated. The cells were plated onto R2 plates using about 3 to 4 ml of R2 overlay per plate. The R2 media was supplemented with 150 $\mu$g/ml tyrosine for identification of mel⁺ and mel⁻ transformants. The regeneration plates contained about 100 $\mu$l of the protoplast-DNA-PEG 1000 solution per plate.

The plates were incubated at 30°C overnight; the following day, the plates were overlayed with R2 overlays containing enough thiostrepton to give a final concentration of 25 $\mu$g/ml after diffusion. Incubation at 30°C was continued; those transformants possessing an intact tyrosinase (mel) gene became black after growth in the presence of tyrosine.

Thiostrepton-resistant, white transformants were isolated, and a number of single colonies were used to inoculate 10 ml TSB cultures containing thiostrepton (25 $\mu$g/ml). The cultures were homogenized and then grown overnight at 30°C in a rotary shaker.

Plasmid isolation for analysis was in accordance with the procedures described in Example 2A; the CsCl gradients of Example 2A were replaced by ethanol precipitations. The mycelium was collected by centrifugation, washed twice with 10.3% sucrose, and then suspended in 1-2 ml of 10.3% sucrose. Four hundred $\mu$l of the cell mixture were transferred to a small tube, and 100 $\mu$l of lysozyme solution were added. The suspension was incubated at 30°C for 30-60 minutes, followed by the addition and mixing of 300 $\mu$l of 0.3 M NaOH containing 1% SDS. The latter solution was kept at 50°C before its addition to the cell mix. The cell mixture was placed at 80°C for 10 minutes, cooled to room temperature, and then extracted with 200 $\mu$l of phenol:CHCl₃ (50:50). The aqueous phase was transferred to a clean tube, made 0.3 M in NaOAc, and then one volume of isopropanol was added. The DNA is incubated at room temperature for five minutes and then pelleted by centrifugation. The pellet was dissolved in 400 $\mu$l of TE buffer and made 0.3 M in NaOAc. About 2.5 volumes of ethanol were added, and the mixture was incubated at -70°C for 30 minutes. After centrifugation and another precipitation, the plasmid DNA was suspended in 50 μl of TE buffer. Restriction enzyme cutting and electrophoretic analysis of the reaction products were used to determine plasmid structure. A variety of different plasmids, each containing a different SphI restriction fragment of phage FP43 were isolated by this procedure.

## B.    Transformation of Streptomyces griseofuscus C581 (ATCC 23916)

The plasmids isolated in Example 4A were used to transform Streptomyces griseofuscus C581. A 10 ml overnight culture of S. griseofuscus was prepared as described for S. lividans in Example 4A. The culture was collected by centrifugation, washed with 3 ml of P media, and resuspended in 3 ml of P media containing 5 to 10 mg/ml of lysozyme. The cells were then incubated at 4°C for one hour, collected by centrifugation, washed twice with 3 ml of P media, and resuspended in 3 ml of P media. For each plasmid prepared in Example 4A (about 0.5 μg in 10 μl of TE buffer), about 150 μl of protoplasts were added to the solution of plasmid DNA. Then, about 100 μl of 55% PEG 1000 and 1 ml of P media were added to each protoplast-DNA mixture. The cells were plated and overlayed with thiostrepton as described in Example 4A.

C. Preparation of Lysates and Transduction

For each plasmid, several transformants obtained in Example 4B were used to prepare lysates using phage FP43 as described in Example 1. These lysates were then used to transduce Streptomyces griseofuscus C581. Transduction was carried out by first obtaining an overnight culture of S. griseofuscus C581, homogenizing and sonicating that culture, and removing several 100 μl aliquots. To each aliquot was added 100 μl of one of the lysates, both directly and after serial dilution, and the mixture was plated on R2 agar using R2 overlays. Thiostrepton was added via an overlay at least 6 hours after plating. The lysate that yielded the greatest number of thiostrepton-resistant transductants contained plasmid pRHB101 packaged into infective FP43 phage particles.

Example 5

Preparation of Transductants

The procedure for transducing Streptomyces griseofuscus described in Example 4C is generally applicable to the actinomycetes. Essentially, transduction merely requires mixing a culture of the organism to be transduced with a transducing lysate and plating the resulting mixture. If FP43 antiserum is used in the transduction, the antiserum should be added about 3 to 6 hours after the cells are plated. It is convenient to add the antiserum to the plates using an overlay. If the antiserum is prepared in accordance with the procedure described in Example 6, only about 3 μl of the antiserum are added per transformation plate. It is convenient to add the thiostrepton to the plates in the same overlay that contains the antiserum.

Example 6

Preparation of Rabbit Polyclonal Antisera to Bacteriophage FP43

Bacteriophage FP43 was used as a suspension in 20 mM Tris-HCl, 4 mM Ca(NO$_3$)$_2$, pH = 8.0. Aliquots of these suspensions were then used as part of a multiple emulsion adjuvant mixture to immunize each of four New Zealand White rabbits.

All immunizations were carried out using a multiple (double) emulsion prepared from a water-in-oil emulsion of the bacteriophage suspension. From $1.32 \times 10^{10}$ to $3.44 \times 10^{10}$ phage were administered to each rabbit in the multiple emulsion on each of six occasions throughout the course of the 16 week immunization schedule.

Bacteriophage were supplied as suspensions containing from 1 to $1.6 \times 10^{11}$ phage/ml. About 0.858 to 1.17 ml of these suspensions made up to at least 1.0 ml with sterile water were added to 1 ml of adjuvant. Dispersion of the aqueous phage suspension in oil emulsion was made using a Sorvall Omi-Mixer with a micro attachment. All procedures involved in making the multiple emulsion were carried out in an ice water bath. The primary emulsion was made at a speed setting of 5 for 5 minutes increasing to 6 for 4 minutes and finally 7 for 1 minute. The primary disperse phase was checked by microscope to assure an even dispersion of small, uniform/sized droplets of the aqueous phage containing the phage within larger, uniformly sized oil droplets. Also, a small drop of the emulsion was placed on the surface of water. A satisfactory emulsion will not spread. This thick, creamy emulsion was then reemulsified with 2 ml of 2 percent Tween 80 (polyoxethylene sorbitan mono-oleate, Sigma) in saline. The resultant free flowing multiple emulsion was then used to immunize the rabbits. The initial immunization was made using Bacto Adjuvant, complete H37Ra adjuvant (Difco Laboratories) containing 1 mg of killed and dried Mycobacterium tuberculosis H37Ra per ml of the adjuvant mixture of 15 percent arlacel A (mannide monooleate) and 85 percent bayol F (paraffin oil). All subsequent immunizations were made using Bacto adjuvant, incomplete Freund (Difco Laboratories) similar to the complete adjuvant but lacking the Mycobacterium.

All immunizations were made with a total of 0.86 cc of the emulsion. The initial immunization on day 0 was made by intravenous administration of 0.25 ml of the adjuvant; the remaining volume was given intramuscularly. All subsequent immunizations were made by the intramuscular route alone. Beginning with the third immunization, 1 cc of Benadryl (diphenhydramine hydrochloride, Park-Davis), 10 mg, was incorporated with each immunization and the mixture divided between two intramuscular sites.

**Immunization schedule and number of bacteriophage administered:**

| Week | Day | Days Since Last Imm. | Number of Bacteriophage Administered |
|------|-----|----------------------|--------------------------------------|
| 1 | 0 | - | $3.00 \times 10^{10}$ |
| 2 | 9 | 9 | $3.00 \times 10^{10}$ |
| 6 | 35 | 26 | $1.89 \times 10^{10}$ |
| 8 | 49 | 14 | $1.32 \times 10^{10}$ |
| 9 | 62 | 13 | $1.81 \times 10^{10}$ |
| 14 | 93 | 31 | $1.60 \times 10^{10}$ |
| 16 | 105 | 12 | Bleed |

Rabbits were bled from the central artery of the ear 12 days following the last immunization. The blood was allowed to clot at 37°C for 30 minutes and then placed on ice overnight. The clots were centrifuged and the serum removed. The serum constituted the FP43 antiserum used in Example 5.

**Claims**
**Claims for the following Contracting States : BE, CH, DE, FR, GB, IT, LI, NL, SE**

1. A recombinant DNA vector that comprises the hft sequence of phage FP43 obtainable from Streptomyces griseofuscus (FP43) NRRL 18184 conferring High Frequency Transduction.

2. The recombinant DNA vector of Claim 1 that comprises the ~7.8 kb SphI restriction fragment of phage FP43.

3. The recombinant DNA vector of Claim 1 that is plasmid pRHB101 produced by ligating SphI-digested plasmid pIJ702 (ATCC 39155) with the ~7.8 kb SphI fragment of phage FP43, or plasmid pRHB106 (NRRL 18183).

EP 0 281 356 B1

**4.** A method for isolating an hft sequence conferring High Frequency Transduction from an actinophage that comprises:

(1) making a genomic library of the actinophage DNA;
(2) transforming the genomic library into a host that supports actinophage infection;
(3) infecting the host cell with the actinophage and collecting the resulting lysate;
(4) using the lysate to transduce an organism of the family Actinomycetales; and
(5) isolating the vectors that transduce at high frequency in step (4).

**5.** The method of Claim 4, wherein the hft sequence is isolated from a Streptomyces phage.

**6.** The method of Claim 5, wherein the phage is FP43.

**7.** A method of transferring DNA encoding a useful substance into an actinomycetes host cell that comprises transducing the cell with a recombinant DNA vector that comprises:

(1) an hft sequence conferring High Frequency Transduction of an actinophage;
(2) a plasmid origin of replication;
(3) the DNA encoding a useful substance; and
(4) a selectable marker.

**8.** The method of Claim 7, wherein the hft sequence is derived from phage FP43 (NRRL 18184).

**9.** The method of Claim 7, wherein the hft sequence is the ~7.8 kb SphI restriction fragment of phage FP43.

**10.** The method of Claim 7, 8, or 9, wherein the actinomycetes is Streptomyces.

**Claims for the following Contracting States : ES, GR**

**1.** A method for isolating an hft sequence conferring High Frequency Transduction from an actinophage that comprises:

(1) making a genomic library of the actinophage DNA;
(2) transforming the genomic library into a host that supports actinophage infection;
(3) infecting the host cell with the actinophage and collecting the resulting lysate;
(4) using the lysate to transduce an organism of the family Actinomycetales; and
(5) isolating the vectors that transduce at high frequency in step (4).

**2.** The method of claim 1, wherein the hft sequence is isolated from a Streptomyces phage.

**3.** The method of Claim 1 or 2, wherein the phage is FP43 obtainable from Streptomyces griseofuscus - (FP43) NRRL 18184.

**4.** A method of transferring DNA encoding a useful substance into an actinomycetes host cell that comprises transducing the cell with a recombinant DNA vector that comprises:

(1) an hft sequence conferring High Frequency Transduction of an actinophage;
(2) a plasmid origin of replication;
(3) the DNA encoding a useful substance; and
(4) a selectable marker.

**5.** The method of Claim 4, wherein the hft sequence is derived from phage FP43 (NRRL 18184).

**6.** The method of Claim 4, wherein the hft sequence is the ~7.8 kb SphI restriction fragment of phage FP43.

**7.** The method of Claim 4, 5, or 6, wherein the DNA encoding a useful substance is an antibiotic biosynthetic gene.

**8.** The method of Claim 4, 5, or 6, wherein the DNA encoding a useful substance is an antibiotic biosynthetic pathway.

23

**9.** The method of Claim 4, 5, or 6, wherein the actinomycetes is Streptomyces.

**10.** A process for preparing plasmid pRHB106 that comprises isolating the plasmid from Streptomyces griseofuscus/pRHB106, NRRL 18103.

**11.** A process for preparing plasmid pRHB101 that comprises ligating SphI-digested plasmid pIJ702 (ATCC 39155) with the ~7.8 kb SphI fragment of phage FP43 (NRRL 18184).

**Patentansprüche**

**Patentansprüche für folgende Vertragsstaaten : BE, CH, DE, FR, GB, IT, LI, NL, SE**

**1.** Rekombinanter DNA Vektor, der die von Streptomyces griseofuscus (FP 43) NRRL 18184 erhältliche und äußerst häufige Transduktion vermittelnde hft-Sequenz des Phagen FP43 enthält.

**2.** Rekombinanter DNA Vektor nach Anspruch 1, der das ~7,8 kb SphI Restriktionsfagment des Phagen FP43 enthält.

**3.** Rekombinanter DNA Vektor nach Anspruch 1, der das durch Ligation des SphI-verdauten Plasmids pIJ702 (ATCC 39155) mit dem ~7,8 kb SphI Fragment des Phagen FP43 hergestellte Plasmid pRHB101 ist oder das Plasmid pRHB106 (NRRL 18183) ist.

**4.** Verfahren zur Isolierung einer eine äußerst häufige Transduktion vermittelnde hft-Sequenz aus einem Actinophagen, gekennzeichnet durch:
   (1) Herstellen einer Genbank aus der Actinophagen DNA,
   (2) Transformieren der Genbank in einen Wirt, der eine Infektion mit Actinophagen zuläßt,
   (3) Infizieren der Wirtszelle mit dem Actinophagen und Gewinnen des entstehenden Lysats,
   (4) Verwenden des Lysats zur Transduktion eines Organismus der Familie der Actinomycetales und
   (5) Isolieren des Vektors, der in Schritt (4) mit hoher Häufigkeit transduziert.

**5.** Verfahren nach Anspruch 4, worin die hft-Sequenz aus einem Streptomyces Phagen isoliert wird.

**6.** Verfahren nach Anspruch 5, worin der Phage FP43 ist.

**7.** Verfahren zum Transferieren einer eine nützliche Substanz kodierenden DNA in eine Actinomycetenwirtszelle, gekennzeichnet durch Transduktion der Zelle mit einem rekombinanten DNA Vektor, der enthält:
   (1) eine hft-Sequenz, die eine äußerst häufige Transduktion eines Actinophagen vermittelt
   (2) einen Replikationsursprung für ein Plasmid,
   (3) die DNA, die eine nützliche Substanz kodiert und
   (4) einen selektierbaren Marker.

**8.** Verfahren nach Anspruch 7, worin die hft-Sequenz vom Phagen FP43 (NRRL 18184) stammt.

**9.** Verfahren nach Anspruch 7, worin die hft-Sequenz das ~7,8 kb SphI Restriktionsfragment des Phagen FP43 ist.

**10.** Verfahren nach Anspruch 7, 8 oder 9, worin der Actinomycet Streptomyces ist.

**Patentansprüche für folgende Vertragsstaaten : GR, ES**

**1.** Verfahren zur Isolierung einer eine äußerst häufige Transduktion vermittelnde hft-Sequenz aus einem Actinophagen, gekennzeichnet durch:
   (1) Herstellen einer Genbank aus der Actinophagen DNA,
   (2) Transformieren der Genbank in einen Wirt, der eine Infektion mit Actinophagen zuläßt,
   (3) Infizieren der Wirtszelle mit dem Actinophagen und Gewinnen des entstehenden Lysats,
   (4) Verwenden des Lysats zur Transduktion eines Organismus der Familie der Actinomycetales und
   (5) Isolieren des Vektors, der in Schritt (4) mit hoher Häufigkeit transduziert.

**2.** Verfahren nach Anspruch 1, worin die hft-Sequenz aus einem Streptomyces Phagen isoliert wird.

**3.** Verfahren nach Anspruch 1 oder 2, worin der aus Streptomyces griseofuscus (FP43) NRRL 18184 erhältliche Phage FP43 ist.

**4.** Verfahren zum Transferieren einer eine nützliche Substanz kodierenden DNA in eine Actinomycetenwirtszelle, gekennzeichnet durch Transduktion der Zelle mit einem rekombinanten DNA Vektor, welcher enthält:

(1) eine hft-Sequenz, die eine äußerst häufige Transduktion eines Actinophagen vermittelt
(2) einen Replikationsursprung für ein Plasmid,
(3) die DNA, die eine nützliche Substanz kodiert und
(4) einen selektierbaren Marker.

**5.** Verfahren nach Anspruch 4, worin die hft-Sequenz vom Phagen FP43 (NRRL 18184) stammt.

**6.** Verfahren nach Anspruch 4, worin die hft-Sequenz das ~7,8 kb SphI Restriktionsfragment des Phagen FP43 ist.

**7.** Verfahren nach Anspruch 4, 5 oder 6, worin die, eine nützliche Substanz kodierende DNA, ein Antibiotikum-biosynthetisierendes Gen ist.

**8.** Verfahren nach Anspruch 4, 5 oder 6, worin die eine nützliche Substanz kodierende DNA ein Antibiotikum-biosynthetisierender Stoffwechselweg ist.

**9.** Verfahren nach Anspruch 4, 5 oder 6, worin der Actinomycet Streptomyces ist.

**10.** Verfahren zur Präparation des Plasmids pRHB106, gekennzeichnet durch Isolierung des Plasmids aus Streptomyces griseofuscus/pRHB106 NRRL 18183.

**11.** Verfahren zur Herstellung des Plasmids pRHB101, gekennzeichnet durch Ligation des SphI-verdauten Plasmids pIJ702 (ATCC 39155) mit dem ~7,8 kb SphI Fragment des Phagen FP43 (NRLL 18184).

**Revendications**

**Revendications pour les Etats contractants suivants : BE, CH, DE, FR, GB, IT, LI, NL, SE**

**1.** Vecteur d'ADN recombinant qui comprend la séquence hft du phage FP43 que l'on peut obtenir à partir de Streptomyces griseofuscus (FP43) NRRL 18184 conférant une transduction haute fréquence.

**2.** Vecteur d'ADN recombinant selon la revendication 1, qui comprend le fragment de restriction SphI d'environ 7,8 kb du phage FP43.

**3.** Vecteur d'ADN recombinant selon la revendication 1, à savoir
le plasmide pRHB101 obtenu en procédant à la ligation du plasmide pIJ702 (ATCC 39155) mis à digérer par SphI avec le fragment SphI d'environ 7,8 kb du phage FP43 ou
le plasmide pRHB106 (NRRL 18183).

**4.** Procédé pour isoler d'un actinophage une séquence hft conférant une transduction haute fréquence, qui comprend le fait de :

(1) fabriquer une librairie génomique de l'ADN actinophagique;
(2) transformer la librairie génomique en un hôte qui supporte l'infection actinophagique;
(3) infecter la cellule hôte avec l'actinophage et recueillir le lysat résultant;
(4) utiliser le lysat pour réaliser la transduction d'un organisme de la famille des Actinomycetales; et
(5) isoler les vecteurs qui réalisent une transduction haute fréquence à l'étape (4).

**5.** Procédé selon la revendication 4, dans lequel la séquence hft est isolée d'un phage Streptomyces.

**6.** Procédé selon la revendication 5, dans lequel le phage est FP43.

**7.** Procédé pour transférer de l'ADN encodant une substance utile dans une cellule hôte actinomycète, qui comprend le fait de réaliser la transduction de la cellule à l'aide d'un vecteur d'ADN recombinant qui comprend :

(1) une séquence hft conférant une transduction haute fréquence d'un actinophage;
(2) une origine plasmidique de réplication;
(3) l'ADN encodant une substance utile; et
(4) un marqueur sélectionnable.

**8.** Procédé selon la revendication 7, dans lequel la séquence hft dérive du phage FP43 (NRRL 18184).

**9.** Procédé selon la revendication 7, dans lequel la séquence hft est le fragment de restriction SphI d'environ 7,8 kb du phage FP43.

**10.** Procédé selon la revendication 7, 8 ou 9, dans lequel l'actinomycète est Streptomyces.

**Revendications pour les Etats contractants suivants : ES, GR**

**1.** Procédé pour isoler d'un actinophage une séquence hft conférant une transduction haute fréquence, qui comprend le fait de :

(1) fabriquer une librairie génomique de l'ADN actinophagique;
(2) transformer la librairie génomique en un hôte qui supporte l'infection actinophagique;
(3) infecter la cellule hôte avec l'actinophage et recueillir le lysat résultant;
(4) utiliser le lysat pour réaliser la transduction d'un organisme de la famille des Actinomycetales; et
(5) isoler les vecteurs qui réalisent une transduction haute fréquence à l'étape (4).

**2.** Procédé selon la revendication 1, dans lequel la séquence hft est isolée d'un phage Streptomyces.

**3.** Procédé selon la revendication 1 ou 2, dans lequel le phage est FP43 que l'on peut obtenir à partir de Streptomyces griseofuscus (FP43) NRRL 18184.

**4.** Procédé pour transférer de l'ADN encodant une substance utile dans une cellule hôte actinomycète, qui comprend le fait de réaliser la transduction de la cellule à l'aide d'un vecteur d'ADN recombinant qui comprend :

(1) une séquence hft conférant une transduction haute fréquence d'un actinophage;
(2) une origine plasmidique de réplication;
(3) l'ADN encodant une substance utile; et
(4) un marqueur sélectionnable.

**5.** Procédé selon la revendication 4, dans lequel la séquence hft dérive du phage FP43 (NRRL 18184).

**6.** Procédé selon la revendication 4, dans lequel la séquence hft est le fragment de restriction SphI d'environ 7,8 kb du phage FP43.

**7.** Procédé selon la revendication 4, 5 ou 6, dans lequel l'ADN encodant une substance utile est un gène biosynthétique d'antibiotique.

**8.** Procédé selon la revendication 4, 5 ou 6, dans lequel l'ADN encodant une substance utile est une voie biosynthétique d'antibiotique.

**9.** Procédé selon la revendication 4, 5 ou 6, dans lequel l'actinomycète est Streptomyces.

**10.** Procédé pour préparer le plasmide pRHB106, qui comprend le fait d'isoler le plasmide à partir de Streptomyces griseofuscus/pRHB106, NRRL 18183.

**11.** Procédé pour préparer le plasmide pRHB101, qui comprend le fait de procéder à la ligation du plasmide pIJ702 (ATCC 39155) mis à digérer par SphI avec le fragment SphI d'environ 7,8 kb du phage FP43 (NRRL 18184).

# FIG.I

## Restriction Site and Function Map of Plasmid pIJ702 (5.65 kb)

# FIG.2

## Restriction Site and Function Map of
## Plasmid pRHB101
## (13.45 kb)

# FIG.3

## Restriction Site and Function Map of Plasmid pRHB106 (13.45 kb)

# Fig. 4

**Effects of Bacteriophage FP43 Concentration on Transduction**